# EUROPEAN PATENT SPECIFICATION

(11) **EP 1 132 100 B1**
(45) Date of publication and mention of the grant of the patent: **15.02.2006**
(21) Application number: 01105944.1
(22) Date of filing: 09.03.2001
(51) Int. Cl.: A61L 27/34, A61F 2/00, A61L 33/00

(54) **A method of treatment of medical-surgical prostheses made of synthetic resins for humans or animals**
Verfahren zur Behandlung von medizinisch-chirurgischen Prothesen aus synthetischen Harzen für Menschen oder Tiere
Méthode de traitement de prothèses médico-chirurgicales de résines synthétiques pour humains ou animaux

(30) Priority: 09.03.2000 IT TO000218
(43) Date of publication of application: 12.09.2001
(73) Proprietor: Lusso, Dario, 10093 Collegno (Torino) (IT); Pepe, Roberto Raffaele, 10122 Torino (IT); Torrisi, Elisabetta, 20041 Agrate Brianza (Milano) (IT); Technoscience S.a.s di Liliana BORGHI & C., 20090 Vimodrone (Milano) (IT); CELTICA IMPIANTI S.R.L., 20122 Milano (IT)
(72) Inventor: Pepe, Roberto Raffaele, 10122 Torino (IT); Lusso, Dario, 10093 Collegno (Torino) (IT); Borghi, Liliana, 20090 Vimodrone (Milano) (IT); Torrisi, Elisabetta, 20041 Agrate Brianza (Milano) (IT)
(74) Representative: Quinterno, Giuseppe

(56) References cited:
- EP-A- 0 348 969
- EP-A- 0 497 204
- US-A- 4 261 806
- US-A- 5 080 924

## Description

The present invention relates to a process for treating medical-surgical prostheses for humans or animals and made of synthetic resins (polymers, elastomers and the like), in order to improve biological compatibility with the tissues of the organisms intended to receive them, and more specifically to a process of the kind defined in the preamble of claim 1.

In the domain of dental prostheses, it is common in the art to treat the prosthesis superficially by immersing it in cleansing liquid or cleaning the surface with dentifrice, in order to improve the superficial "wettability" of the prosthesis and to form a so-called barrier film to protect the oral cavity against allergens from the prosthesis.

In the domain of ophthalmic prostheses it is likewise usual to treat the prostheses with a disinfectant liquid, or with an oil-based liquid or paste operable to alter the wettability thereof.

The effect of such treatments is brief, however, and must be carried out fairly frequently.

Other types of medical-surgical prostheses made of synthetic resins, either for humans or for animals, can also pose problems of allergy over time, or of intolerance on the part of adjacent tissues, or of infiltration by foreign organic agents or the like. These side effects are linked to the surface characteristics of polymeric resins, in particular to the presence of residual monomers or to surface porosity of the prosthesis itself.

A process of the initially defined kind is disclosed in US-A-5 080 924. This document describes plasma-induced grafting of vaporised hexamethyldisiloxane monomers on a previously etched surface of a prosthesis, preferably an intraocular lens, where the etching is also carried out by a plasma treatment using gases such as argon, ammonia, nitrogen or oxygen (the latter being less preferred). The monomers polymerise on the surface. Plasma treatment (both etching and grafting) occurs at 40°-50°C, 0.1-0.6 torr=0.133-1 mbar, for 1 to 30 min with an energy of 10-220 Watt. The plasma generator uses radio frequency (microwave) operating at 13.56 MHz.

US-A-4 261 806 discloses a process which combines hexamethyldisiloxane and argon in a ratio of 1:1 to plasma-graft organopolysiloxanes to the inner surface of a polymeric tube useful as a medical device, e.g. artificial vascular tracts or esophagi. The plasma generation is carried out at low temperature and vacuum and can be realised by any standard plasma generation technique such as DC, radio frequency or microwave. As alternative for argon oxygen is mentioned.

The object of the present invention is to provide a process for plasma-treating dental or ophtalmic prostheses of synthetic resin, either for humans or for animals, which makes it possible to improve the overall biological compatibility of a prosthesis with the tissues of the receiving organisms, and which has an (almost) permanent effect, thereby eliminating or dramatically reducing the need for repeat treatments.

This and other objects are achieved according to the invention by providing a treatment for prostheses of which the main characteristics are defined in the appended Claim 1.

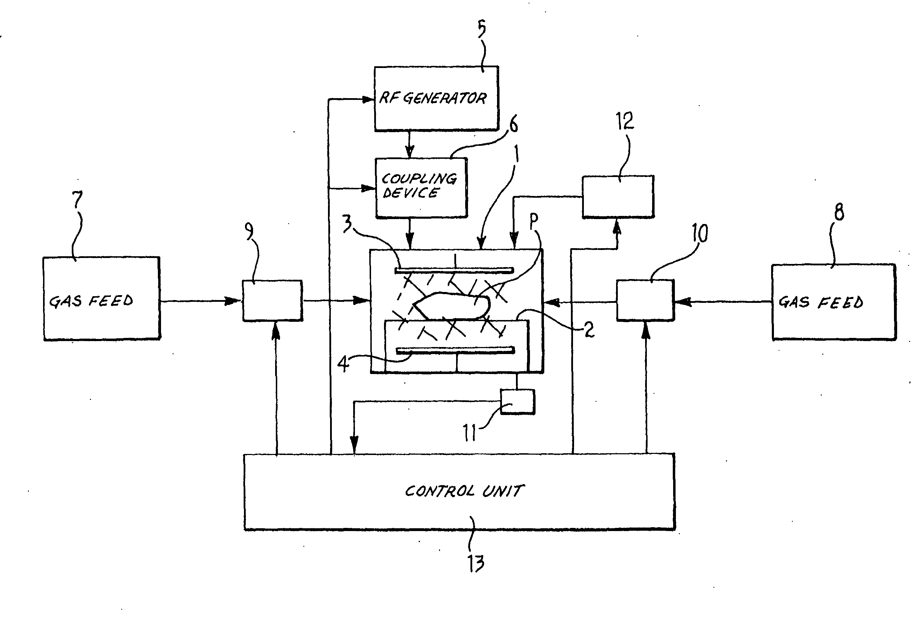

Further characteristics and advantages of the invention will become apparent from the detailed description which follows, provided purely by way of non-limitative example, with reference to the appended drawing which is a block diagram of apparatus to be used for carrying out the process of the invention.

In the drawing, an environment or chamber for carrying out the treatment of the invention on a prosthesis P of synthetic resin, for example a dental prosthesis such as a set of false teeth, or an ophthalmic prosthesis such as a contact lens, is indicated 1.

The prosthesis P is arranged on a support structure 2 in the treatment chamber 1, between two electrodes 3 and 4 which face each other.

The electrodes 3 and 4 are connected to the output of a radio-frequency generator 5 by means of a controllable coupling device 6.

Two gas-supply devices are connected to the treatment chamber 1 by respective flow regulators 9 and 10.

The treatment chamber 1 is also provided with a pressure/depression sensor or transducer 11 and a controllable pressure/depression regulator 12.

The coupling device 6, the gas-flow regulators 9 and 10 and the pressure/depression regulator 12 are controlled according to a predetermined system by an electronic control unit 13, which also receives the signals from the pressure/depression sensor or transducer 11.

The operation of the apparatus shown in the drawing will now be described with reference to an exemplary application of the treatment of the invention to dental or ophthalmic prostheses.

In use, two gases, in particular oxygen and hexamethyldisiloxane, are fed into the chamber 1 by the gas feeds 7 and 8 in a predetermined volume ratio, for convenience in a ratio of substantially 1:2.

The control unit 13 controls the regulator device 12 in dependence on the signals from the sensor or transducer 11, in such a way that the pressure in the treatment chamber 1 is maintained substantially at a predetermined delivery value, at substantially 0.45mbar, for example.

The generator 5 then produces an electric discharge between the electrodes 3 and 4, thereby ionizing the gas mixture in the treatment chamber 1.

For the treatment of dental or ophthalmic prostheses, it is convenient for the generator 5 to have a frequency in the range of 10-15MHz, preferably in the range between 13 and 14MHz, and most conveniently of around 13.56MHz.

The connection between the generator 5 and the treatment chamber 1 is adjusted, by means of the coupling device 6, in such a way that the power transmitted to the area containing the plasma (where the prosthesis is being treated) is around 100W and the reflected power is around 1% or 2% of the power transmitted.

The prosthesis P is exposed to the plasma formed between the electrodes 3 and 4 as an effect of the ionization of the gas mixture.

In the case of dental or ophthalmic prostheses, the most convenient duration of exposure to the plasma is between 30 and 60 seconds, with around one minute being preferable.

The treatment is carried out substantially at ambient temperature (around 300°K).

During exposure to the plasma, the free electrons, ions and metastable particles contained therein collide with the surfaces of the prosthesis P made of polymeric resin, thereby breaking a certain number of chemical bonds in the surface layer of the prosthesis and forming free radicals. These free radicals are then generally subject to additional reactions, in dependence on the composition of the ionized gas.

Exposure of the prosthesis to the plasma generally results in the formation of a surface layer with very different chemical-physical properties from those of the initial polymeric mass. However the effect of this exposure is confined to a thin surface molecular layer.

The surface of a dental prosthesis treated with the process of the invention has been examined with a trinocular optical microscope (X 100) and by Fourier transformation infrared spectroscopy.

The optical microscopic examination was used to determine the morphological characteristics of the surface layer. The surface layer of the treated prosthesis was characterised by "islands" of SiOₓ bonded together by an organic component which is also chemically bonded to the underlying layer.

The spectroscopic analysis revealed absorption bands at 1105.67 and 1025.38 cm⁻¹, attributable to polysiloxanes, which confirm the inorganic nature of the matrix of the surface layer.

An absorption band was also found at 1260.40 cm⁻¹, attributable to symmetrical deformation of the methyl ("bending" of CH₃).

Another absorption band was found at 1244.03 cm⁻¹, attributable to asymmetrical deformation of the methyl ("bending of CH₃), thereby confirming the organic nature of the components binding the islands of SiOₓ.

Finally, absorption bands were detected at 813.64 and 794.748 cm⁻¹, attributable to stretching of the molecules of Si(CH₃)₂.

In general, the gas or gas mixture in the treatment chamber can be ionized by means of electric discharges generated by applying either DC or AC power to electrodes, or by producing discharges by means of microwaves.

By using suitable gases or suitable mixtures of gases, it is possible to achieve surface "cleaning" and/or "activation" effects, by generating chemically active sites on the surface of the prosthesis and using these to anchor, as a result of exposure to the ionized gas, groups or radicals operable to impart characteristics of chemical inertia to the surface of the prosthesis or, at least, dramatically to reduce surface porosity, while leaving the structural characteristics of the prosthesis unchanged and at the same time forming a surface barrier against aggression and infiltration by (organic and/or inorganic) external agents.

It is thus possible to give the prosthesis either hydrophobic or hydrophilic characteristics, according to requirements.

The surface of a prosthesis can be made neutral, that is inert or clean, thereby reducing the ability of organic or inorganic external agents to attack it and, as a result, extending its life and improving its compatibility with the human or animal organism for which the prosthesis is intended.

The process also allows for the treatment of heart, orthopaedic, odontostomatological/orthodontic, auditory, ophthalmic, chest or abdominal, cranial and vascular prostheses to be treated, as well as prostheses used in cosmetic surgery and the like.

In particular, the process makes it possible to cure stomatological pathologies, such as "burning mouth syndrome", xerostomy and opportunist secondary microbial infections, ophthalmic problems (intolerance or allergy to contact lenses) and xerophthalmia.

## Claims

1. A process for treating medical-surgical prostheses (P) for humans or animals and made of synthetic resin, which includes the steps of:
providing a treatment environment or chamber (1);
circulating a flow of gas or a mixture of gases through the said environment or chamber;
causing the gas or gas mixture in the said environment or chamber (1) to be ionized so as to form plasma; and
exposing the prosthesis (P) to be treated to the said plasma;
the process being **characterised in that** a mixture of oxygen and hexamethyldisiloxane is used for treatment of dental or ophthalmic prostheses, said mixture containing oxygen and hexamethyldisiloxane in a volumetric ratio of substantially 1:2.

2. A process according to Claim 1, in which the gas or gas mixture is ionized by means of electric discharges produced by applying either DC or AC power between at least two electrodes (3, 4) arranged in the treatment environment or chamber (1).

3. A process according to Claim 1, in which the gas or gas mixture is ionized by producing electric discharges in the said treatment environment or chamber (1) by means of a microwave source (5).

4. A process according to any preceding Claim, in which the pressure of the gas or gas mixture in the said treatment environment or chamber (1) is substantially maintained at a predetermined value, lower than atmospheric pressure.

5. A process according to Claim 1, in which the gas mixture in the treatment environment or chamber (1) is kept at around 0.45mbar.

6. A process according to Claim 5, in which the prosthesis (P) is exposed to the plasma for a period of time of between 30 and 100 seconds, and preferably for around 1 minute.

7. A process according to Claim 1 and any subsequent Claim, in which the gas mixture is ionized by a source of radio frequency (5) operable to generate a frequency in the range of 10-15MHz, and preferably operating at a frequency of between 13 and 14 MHz.

8. A process according to Claim 7, in which a source of radio frequency is used, operable to generate an electric discharge of around 100W in the treatment chamber (1).

## Patentansprüche

1. Verfahren zum Behandeln medizinisch-chirurgischer Prothesen (P) für Menschen oder Tiere, die aus Kunstharz gefertigt sind, welches die folgenden Schritte umfasst:
das Bereitstellen einer Behandlungsumgebung oder -kammer (1);
das Leiten eines Gasstroms oder einer Mischung von Gasen durch die Umgebung oder Kammer;
das Bewirken einer Ionisierung des Gases oder der Gasmischung in der Umgebung oder Kammer (1) zwecks Bildung von Plasma; und
das Aussetzen der zu behandelnden Prothese (P) an das Plasma;
wobei das Verfahren **dadurch gekennzeichnet ist, dass** eine Mischung von Sauerstoff und Hexamethyldisiloxan für die Behandlung von Zahn- oder Augenprothesen verwendet wird, wobei diese Mischung Sauerstoff und Hexamethyldisiloxan in einem Volumenverhältnis von im Wesentlichen 1:2 enthält.

2. Verfahren gemäß Anspruch 1, wobei das Gas oder die Gasmischung durch elektrische Entladungen ionisiert wird, die erzeugt werden, indem entweder Gleichstrom oder Wechselstrom zwischen zumindest zwei in der Behandlungsumgebung oder -kammer (1) angeordneten Elektroden (3, 4) angelegt wird.

3. Verfahren gemäß Anspruch 1, wobei das Gas oder die Gasmischung ionisiert wird, indem in der Behandlungsumgebung oder -kammer (1) elektrische Entladungen mittels einer Mikrowellenquelle (5) erzeugt werden.

4. Verfahren gemäß irgendeinem vorhergehenden Anspruch, wobei der Druck des Gases oder der Gasmischung in der Behandlungsumgebung oder-kammer (1) im Wesentlichen bei einem vorbestimmten Wert, der niedriger als der Atmosphärendruck ist, gehalten wird.

5. Verfahren gemäß Anspruch 1, wobei die Gasmischung in der Behandlungsumgebung oder -kammer (1) bei etwa 0,45 mbar gehalten wird.

6. Verfahren gemäß Anspruch 5, wobei die Prothese (P) für einen Zeitraum zwischen 30 und 100 Sekunden und vorzugsweise etwa 1 Minute lang dem Plasma ausgesetzt wird.

7. Verfahren gemäß Anspruch 1 und einem der nachfolgenden Ansprüche, wobei die Gasmischung durch eine Hochfrequenzquelle (5) ionisiert wird, die dazu betriebsfähig ist, eine Frequenz im Bereich von 10-15 MHz zu erzeugen, und die vorzugsweise bei einer Frequenz zwischen 13 und 14 MHz funktioniert.

8. Verfahren gemäß Anspruch 7, wobei eine Hochfrequenzquelle verwendet wird, die dazu betriebsfähig ist, in der Behandlungskammer (1) eine elektrische Entladung von etwa 100W zu erzeugen.

## Revendications

1. Procédé de traitement de prothèses médico-chirurgicales (P) en résine synthétique, à usage humain ou vétérinaire, qui comprend les étapes où:
On fournit un environnement ou une chambre de traitement (1) ;
On fait circuler un flux d'un gaz ou d'un mélange de gaz dans ledit environnement ou ladite chambre;
On induit une ionisation du gaz ou du mélange gazeux dans ledit environnement ou ladite chambre de traitement (1) de manière à former un plasma ; et
On expose la prothèse (P) à traiter audit plasma ;
le procédé étant **caractérisé en ce qu'**un mélange d'oxygène et d'hexaméthyldisiloxane est utilisé pour le traitement de prothèses dentaires ou ophtalmiques, ledit mélange contenant de l'oxygène et de l'hexaméthyldisiloxane dans un ratio volumétrique sensiblement égal à 1 : 2.

2. Procédé selon la revendication 1, dans lequel le gaz ou le mélange de gaz est ionisé au moyen de décharges électriques produites en appliquant un courant continu ou alternatif entre au moins deux électrodes (3, 4) disposées dans l'environnement ou la chambre de traitement (1).

3. Procédé selon la revendication 1, dans lequel le gaz ou le mélange gazeux est ionisé par émission de décharges électriques dans ledit environnement ou ladite chambre de traitement (1) au moyen d'une source de micro-ondes (5).

4. Procédé selon l'une quelconque des revendications précédentes, dans lequel la pression du gaz ou du mélange gazeux dans ledit environnement ou ladite chambre de traitement (1) est pratiquement maintenue à une valeur prédéterminée, inférieure à la pression atmosphérique.

5. Procédé selon la revendication 1, dans lequel le mélange gazeux dans ledit environnement ou ladite chambre de traitement (1) est maintenu à une pression de l'ordre de 0,45 mbar.

6. Procédé selon la revendication 5, dans lequel la prothèse (P) est exposée au plasma pendant une durée comprise entre 30 et 100 secondes, et de préférence pendant environ 1 minute.

7. Procédé selon la revendication 1 et l'une quelconque des revendications suivantes, dans lequel le mélange gazeux est ionisé par une source de radiofréquence (5) capable de fonctionner pour fournir une fréquence dans une gamme de 10 à 15 MHz, et de préférence fonctionnant à une fréquence comprise entre 13 et 14 MHz.

8. Procédé selon la revendication 7, dans lequel on utilise une source de radiofréquence, capable de fonctionner pour fournir une décharge électrique d'environ 100 W dans la chambre de traitement (1).
